# FASCICULE DE BREVET EUROPEEN

(11) **EP 2 089 374 B1**
(45) Date de publication et mention de la délivrance du brevet: **04.08.2010**
(21) Numéro de dépôt: 07870250.3
(22) Date de dépôt: 06.11.2007
(51) Int. Cl.: C07D 327/02, C07C 323/20

(54) **PROCEDE DE SYNTHESE DU N- [3- [ (2-METHOXYPHENYL) SULFANYL] -2- METHYLPROPYL] -3,4-DIHYDRO-2H-1,5-BENZOXATHIEPIN-3-AMINE**
VERFAHREN ZUR SYNTHESE VON N-[3-[(2-METHOXYPHENYL)SULFANYL-2-METHYLPROPYL-3,4-DIHYDRO-2H-1,5-BENZOXATHIEPIN-3-AMIN
METHOD FOR THE SYNTHESIS OF N- [3- [ (2-METHOXYPHENYL) SULFANYL] -2- METHYLPROPYL]-3,4-DIHYDRO-2H-1,5-BENZOXATHIEPIN-3-AMINE

(30) Priorité: 08.11.2006 FR 0609815
(43) Date de publication de la demande: 19.08.2009
(73) Titulaire: Pierre Fabre Medicament, 92100 Boulogne-Billancourt (FR)
(72) Inventeur: VACHER, Bernard, 81100 Castres (FR); BRUNEL, Yves, 81150 Marssac-Sur-Tarn (FR); MAUREL, Jean-Louis, 81100 Burlats (FR)
(74) Mandataire: Rousseau, Pierrick Edouard
(86) Numéro de dépôt international: PCT/FR2007/001831
(87) Numéro de publication internationale: WO 2008/068403

(56) Documents cités:
- WO-A-2005/103027

## Description

La présente invention concerne un nouveau procédé de préparation du dérivé de formule (1) 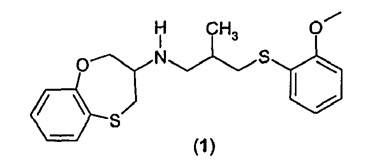 ses sels d'addition et les hydrates de ces sels d'addition avec les acides minéraux ou les acides organiques pharmaceutiquement acceptables, ainsi que leurs formes tautomères, les énantiomères, les mélanges d'énantiomères et les stéréoisomères purs ou en mélange racémique ou non.

Le nouveau procédé s'applique de préférence au composé (1) dont l'atome de carbone stéréogénique du fragment 3,4-dihydro-2H-1,5-benzoxathiépine est de configuration absolue (R) et celui de la chaîne propyle est de configuration absolue (S). Les descripteurs (R) et (S), utilisés pour spécifier la configuration absolue des atomes stéréogéniques contenus dans la molécule de formule (1), sont définis tels que dans la règle de priorité de Cahn-Ingold-Prelog (E.L. Eliel and S.H. Wilen Stereochemistry of Organic Compounds, John Wiley & Sons, Inc., chap. 5, 104-12, 1994).

Dans un mode particulièrement avantageux de réalisation de l'invention, le stéréoisomère du composé (1) choisi est le :
(3R)-N-{(2S)-3-[(2-Méthoxyphényl)sulfanyl]-2-méthylpropyl}-3,4-dihydro-2H-1,5-benzoxathiépin-3-amine ;
ses sels d'addition et les hydrates de ces sels d'addition avec les acides minéraux ou les acides organiques pharmaceutiquement acceptables. Dans la présente invention, un stéréoisomère est considéré comme pur s'il est associé avec moins de 1% d'un autre stéréoisomère ou d'un mélange d'autres stéréoisomères (i.e., excès diastéréoisomérique ≥ 98%, l'actualité chimique 2003, 11/12, 10-4).

Le composé de formule (1), est décrit dans la demande de brevet WO 02/081464, où il est revendiqué comme étant utile dans le traitement de l'angor stable, l'angor instable, l'insuffisance cardiaque, le syndrome du QT long d'origine congénitale, l'infarctus du myocarde et les troubles du rythme cardiaque.

Dans cette demande, le composé de formule (1) est obtenu par amination réductrice entre le composé de formule (2) et l'aldéhyde de formule (5), selon le schéma I suivant :

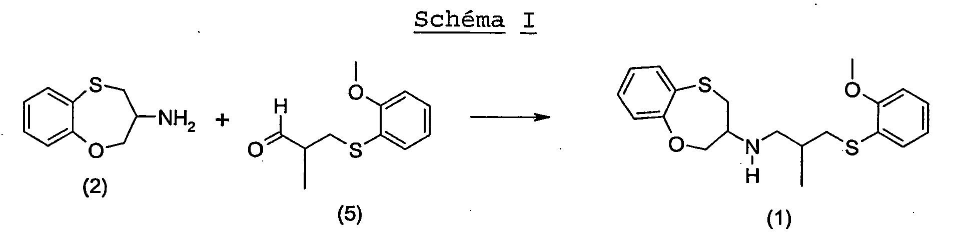

Toutefois, cette réaction d'amination réductrice pose des problèmes en raison de l'instabilité chimique et stéréochimique de l'aldéhyde de formule (5). De plus, de part la nature de certains des réactifs utilisés ainsi que celle de certains des sous-produits formés, cette séquence est difficilement réalisable au plan industriel.

Dans le procédé de synthèse décrit dans la demande WO 05/103027, le composé de formule (1) est préparé selon le schéma II par réduction de l'amide de formule (6), stable chimiquement et stéréochimiquement. L'avantage de ce procédé réside essentiellement dans le fait qu'il est robuste, réalisable au plan industriel, et donc éventuellement utilisable pour la production de larges quantités du composé (1), contrairement au procédé illustré dans WO 02/081464.

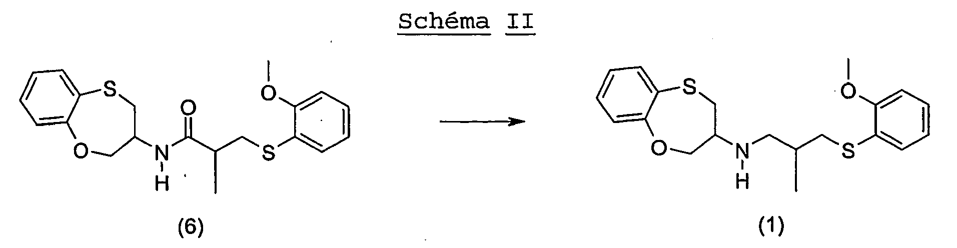

Cependant, bien que ce procédé soit réalisable sur le plan industriel, il comporte deux inconvénients majeurs qui en limitent, ou en compliquent considérablement l'exploitation :
- La dernière étape du procédé WO 05/103027 met en oeuvre la réduction d'une fonction amide en l'amine correspondante au moyen d'un complexe de borane (BH₃.THF) à chaud. Dans les conditions expérimentales requises pour effectuer la dite réaction de réduction, l'hydrure de bore (BH₃) peut engendrer du diborane B₂H₆, toxique, extrêmement inflammable et explosif (INRS, FT n°188, 1987). En raison des risques potentiel d'intoxication, d'incendie et d'explosion dus à la formation éventuelle de B₂H₆, des mesures sévères de prévention et de protection doivent être mises en place lors de la manipulation du complexe BH₃.THF, ce qui complique considérablement l'exploitation du procédé.
   Par ailleurs, il s'avère que la réduction de l'amide (6) en l'amine (1) au moyen d'agents réducteurs autres qu'un complexe de borane (i.e., ne pouvant pas engendrer du B₂H₆) n'est pas satisfaisante.
- Un autre facteur limitant provient du fait que le procédé décrit dans WO 05/103027 fait intervenir comme matière première, dans la synthèse du composé intermédiaire (6) le méthyl (R)-3-hydroxy-2-méthyl-propanoate [72657-23-9], difficilement accessible à l'échelle industrielle.

La mise au point d'un procédé de synthèse réalisable industriellement sans les inconvénients décrits précédemment est donc nécessaire pour la préparation de médicaments utiles dans de traitement de l'angor stable, l'angor instable, l'insuffisance cardiaque, le syndrome du QT long d'origine congénitale, l'infarctus du myocarde et les troubles du rythme cardiaque.

L'objet de la présente invention concerne donc un nouveau procédé de synthèse du composé (1) exempt des limitations exposées ci-dessus.

Ainsi, le procédé de l'invention, contrairement à celui illustré dans WO 05/103027, ne fait plus intervenir la réduction d'une fonction amide impliquant la mise en oeuvre d'un agent réducteur du type complexe de borane. La sécurité du nouveau procédé s'en trouve considérablement accrue.

Une autre amélioration importante du procédé de l'invention se situe au niveau de l'utilisation d'un dérivé du type (S)-3-halogeno-2-methylpropanol comme matière première plus accessible à large échelle que le méthyl (R)-3-hydroxy-2-méthyl-propanoate utilisé dans le procédé décrit dans WO 05/103027.

Donc, sur le plan de la sécurité d'exploitation et sur le plan économique, le procédé de l'invention comporte des avantages marqués par rapport au procédé décrit dans la demande de brevet WO 05/103027.

Un premier aspect de l'invention concerne donc l'amélioration du procédé de synthèse du composé de formule (1)

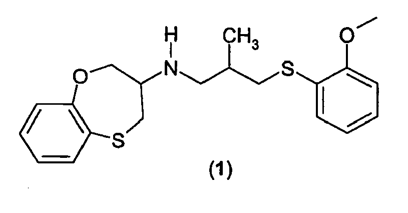

Ce composé est obtenu par condensation des composés de formule (2) et (3) 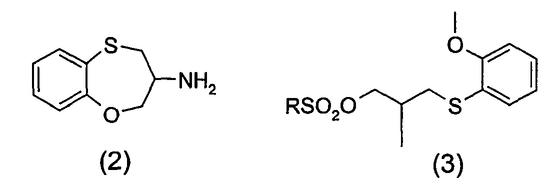 dans laquelle le radical R représente un groupe méthyle ou, de préférence, un groupement 4-méthylphényl, selon le schéma III.

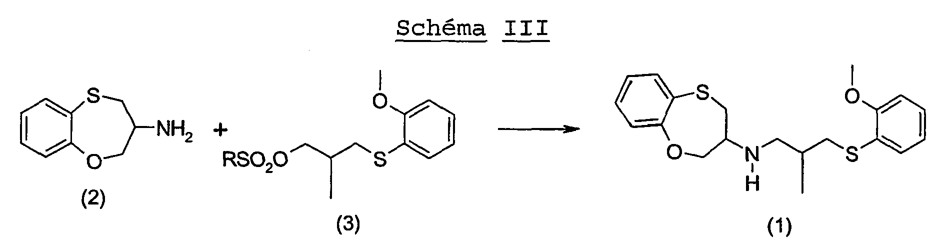

Le stéréoisomère préféré des composés de formule (1), (2) et (3) est, dans tous les cas, celui dont les atomes de carbone stéréogéniques du fragment 3,4-dihydro-2H-1,5-benzoxathiépine et de la chaîne sont de configuration absolue (R) et (S), respectivement.

Selon le nouveau procédé de l'invention, l'amine (2) réagit thermiquement, de préférence entre 100-200°C, sur un dérivé de formule (3), de préférence le tosylate, en présence d'une base, par exemple une base azotée non nucléophile telle que la diisopropyléthylamine ou la triéthylamine, dans un solvant inerte tel que, le toluène ou le xylène.

La substitution d'un groupe tosylate par une amine primaire est une réaction bien connue en chimie organique, par exemple Organic Process Research & development 2005, 9(3), 314. Toutefois, de façon inattendue, la réaction de l'amine (2) sur le tosylate (3) s'avère remarquablement efficace. En effet, au cours de la dite réaction il n'a pas été observé la formation de produit résultant de di-alkylation de l'amine (2) par le tosylate (3), ni de produits secondaires provenant de l'élimination d'acide p-toluènesulfonique à partir de (3). De plus, dans les conditions de la réaction il n'y a pas de racémisation substantielle, tant au niveau des intermédiaires (2) et (3) qu'au niveau du produit formé (1). Il s'ensuit que le produit de formule (1) est obtenu avec un rendement et une pureté stéréochimique satisfaisants. D'un point de vue technique, la mise en oeuvre de la réaction et le traitement du mélange réactionnel sont faciles à réaliser. Le procédé de l'invention est donc particulièrement adapté à la production de large quantité du composé de formule (1).

Un deuxième aspect de l'invention consiste en un procédé de synthèse du nouvel intermédiaire de formule (3) 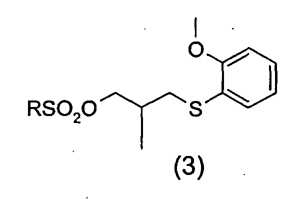 selon le schéma IV 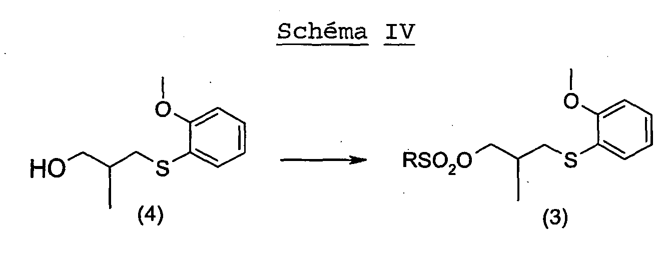 dans lequel le composé de formule (4) : 3-(2-methoxyphenylsulfonyl) 2-methyl propanol 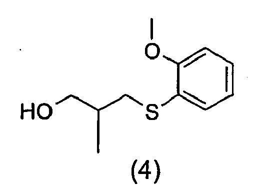 est traité au moyen du chlorure de méthanesulfonyle ou du chlorure de paratoluènesulfonyle, en présence d'une base organique ou minérale, telles que par exemple, la pyridine, la triethylamine, du carbonate de potassium ou la potasse pilée anhydre, pour donner le composé de formule (3). Ainsi, la fonction alcool du 3-(méthoxyphényl)sulfanyl-2-méthylpropanol de formule (4) est activée, de préférence sous la forme d'un tosylate, pour donner le nouveau composé de formule (3). L'énantiomère préféré des composés de formule (3) est celui dont l'atome de carbone stéréogénique est de configuration absolue (S).

Le composé de formule (3) est ensuite condensé avec l'amine (2) pour donner le composé (1) (schéma III).

Un autre aspect de l'invention concerne le composé intermédiaire de formule générale (3) 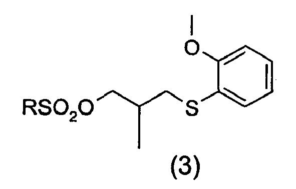 dans laquelle le radical R est un groupe méthyle ou préférentiellement 4-méthylphenyl.

Plus particulièrement, l'invention concerne le composé intermédiaire de formule générale (3) tel que décrit précédemment dans lesquels, l'atome de carbone stéréogénique de la chaîne propyle est de configuration absolue (S)

Les exemples suivants illustrent l'invention sans toutefois en limiter la portée.

### Exemple 1 : (S)-3-(2-méthoxyphényl)sulfanyl-2-méthylpropanol, 4-methylbenzenesulfonate (3)

Dans un tricol sous atmosphère inerte on introduit 10ml de chlorure de méthylène, 2g (9,4 mmoles) de (*S*)-3-(2-méthoxyphenylsulfanyl)-2-méthylpropanol, 2g (10,5 mmoles) de chlorure de paratoluènesulfonyle, 115mg (0,94 mmoles) de 4-diméthylaminopyridine et 1,2g (12,1 mmoles) de triéthylamine sont ensuite additionnée goutte à goutte. Après 2 heures d'agitation à température ambiante, 10ml d'eau déminéralisée sont ajoutés. Le pH est ajusté à 6,5 par addition d'acide chlorhydrique 2N. Les phases sont séparées puis la phase organique lavée par 10ml d'eau déminéralisée. La phase organique est concentrée sous pression réduite. L'huile jaune obtenue (3,2g) est reprise dans un mélange de chlorure de méthylène (4,8ml) et 19,2ml d'éther isopropylique. Le précipité obtenu est filtré puis lavé avec 2 fois 10ml d'éther isopropylique et séché à 40°C sous pression réduite. Le produit du titre (3) est obtenus sous la forme d'une poudre blanche 2,6g (75%).
Rf = 0,56 (heptane / AcOEt : 60/40 ; silice Merck 254)
Pf = 86-87°C
¹H RMN (CDCl₃) δ : 1,03 (d, 3H, J=7,0 Hz) ; 2,02 (m, 1H) ; 2,43 (s, 3H) ; 2,71 (dd, 1H, J=6,8 Hz, J=13,0 Hz) ; 2,89 (dd, 1H, J=6,7 Hz, J=13,0 Hz) ; 3,87 (s, 3H) ; 4,01 (d, 2H, J=5,6 Hz) ; 6,84 (d, 1H, J=7,6 Hz) ; 6,88 (dd, 1H, J=7,1 Hz, J=7,6 Hz) ; 7,10 (d, 1H, J=7,6 Hz) ; 7,18 (dd, 1H, J=7,1 Hz, J=7,6 Hz) ; 7,32 (d, 2H, J=8,1 Hz) ; 7,77 (d, 2H, J=8,1 Hz).
¹³C RMN (CDCl₃) δ : 16,26 ; 21,62 ; 32,95 ; 35,14 ; 55,73 ; 73,37 ; 110,60 ; 121,02 ; 123,63 ; 127,62 ; 127,88 (2C) ; 129,81 (2C) ; 130,21 ; 132,88 ; 144,70 ; 157,64.
HPLC, colonne chiralcel OD (250 x 4,6 mm), eluant (hexane/éthanol/diéthylamine : 97/3/0,1), 1 ml/min, Tr (S) = 21,02 min ; Tr (R) = 19,77 min. Pureté chirale (isomère S) déterminée à partir de l'aire sous la courbe (99%).
MS (APCI+) m/z : 367 (M+H), 195 (M-TsO).

### Exemple 2 : (3R)-N-{(2S)-3-[(2-méthoxyphényl)sulfanyl]-2-méthylpropyl}-3,4-dihydro-2H-1,5-benzoxathiépin-3-amine (1)

Dans un tricol sous atmosphère inerte on introduit 6,5g (35,9 mmoles) de (*R*)-3,4-dihydro-2*H*-1,5-benzoxathiepin-3-amine et 11g (30 mmoles) de (*S*)-3-(2-méthoxyphenylsulfanyl)-2-methylpropyl 4-methylbenzenesulfonate dans 33ml de toluène. 6g (60 mmoles) de triéthylamine sont ajoutés puis le milieu réactionnel est chauffé sous agitation à 105°C pendant 24h. Après retour à température ambiante 33ml de toluène sont ajoutés. La phase organique est lavée deux fois par 66ml d'eau déminéralisée puis décolorée par agitation pendant une heure en présence de 2g de Noir CXV. Après concentration sous pression réduite on réalise une chasse avec 33ml d'éthanol 100. Le produit du titre est obtenu sous forme d'une huile jaune (8,85g ; 79%).
Rf = 0,53 (heptane / AcOEt : 60/40 ; silice Merck 254) 8,85g (23,5 mmoles) de composé (1) sont introduits dans 53ml d'un mélange éthanol 100 / méthyl terbutyl ether (2/4 v/v). Une solution d'acide bromhydrique 62% (3g ; 23,5 mmoles) est coulée lentement sur la solution d'amine à température ambiante puis après agitation deux heures à température ambiante le solide obtenu est filtré et lavé par 3 fois 15ml de méthyl terbutyl ether. Un séchage pendant 12 heures à 50°C fourni 7,3g (68%) du bromhydrate du composé (1).
Pf = 129-131°C ;
¹H RMN (DMSO-d₆) ? 1.12 (d, 3H), 2.19 (m, 1H), 2.82 (dd, 1H), 3.05 (lm, 1H), 3.12 (dd, 1H), 3.23 (lm, 1H), 3.32 (m, 2H), 3.82 (s, 3H), 3.90 (1s, 1H), 4.39 (dd, 1H), 4.52 (dd, 1H), 6.98 (m, 2H), 7.07 (m, 2H), 7.23 (m, 3H), 7.40 (dd, 1H), 8.88 (ls, 2H échangeables) ;
HPLC, colonne chiralcel OJ (250 x 4,6 mm), éluant (méthanol / éthanol / diéthylamine : 50/50 0,1) 1 ml/min, Tr (R,S) = 24,37 min ; Tr (R,R) = 16,80 min ; Tr (S,S) = 19,79 min ; Tr (S,R) = 15,05 min). Pureté chirale (isomère R,S) déterminée par le pourcentage d'aire sous la courbe (98%).

## Revendications

1. Procédé de préparation du N-[3-[(2-methoxyphenyl)sulfanyl]-2-methylpropyl]-3,4-dihydro-2H-1,5-benzoxathiepin-3-amine de formule générale (1) 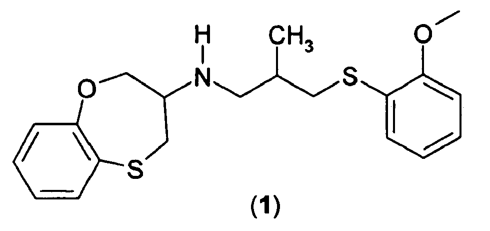 **caractérisé en ce qu'**il met en oeuvre la condensation des intermédiaires de formule (2) et (3) 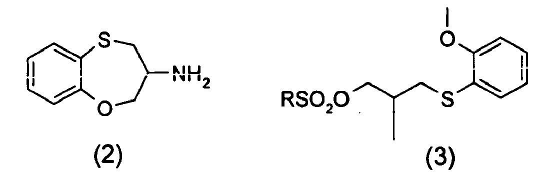 dans laquelle le radical R est un groupe méthyle ou, de préférence, un groupement 4-méthylphényl.

2. Procédé selon la revendication 1 **caractérisé en ce que** les intermédiaires de formule (3), dans lequel R est un groupement méthyl ou de préférence un groupement 4-méthylphényl 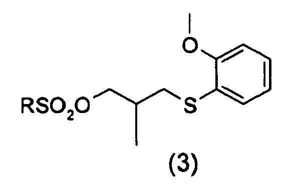 sont préparés par la mise en oeuvre de la réaction du 3-[(2-methoxyphenyl)sulfanyl]-2-méthylpropanol 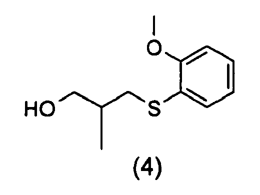 avec un chlorure de sulfonyle approprié pour donner le composé de formule (3)

3. Procédé de préparation du N-[3-[(2-methoxyphenyl)sulfanyl]-2-methylpropyl]-3,4-dihydro-2H-1,5-benzoxathiepin-3-amine de formule (1), selon les revendications 1 à 2, **caractérisé en ce que** le composé (1) est de configuration absolue (R) au niveau de l'atome de carbone stéréogénique du fragment 3,4-dihydro-2H-1,5-benzoxathiépine et de configuration absolue (S) au niveau de l'atome de carbone stéréogénique de la chaîne propyle.

4. Procédé de préparation du N-[3-[(2-methoxyphenyl)sulfanyl]-2-methylpropyl]-3,4-dihydro-2H-1,5-benzoxathiepin-3-amine de formule (1) selon les revendications 1 à 3, **caractérisé en ce que** l'atome de carbone stéréogénique de l'intermédiaire de formule générale (3) utilisé est de configuration absolue (S).

5. Intermédiaires de formule (3) dans laquelle le radical R est un groupe méthyle ou, de préférence, un groupement 4-méthylphényl.

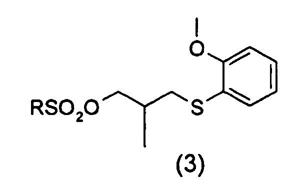

6. Utilisation des intermédiaires de formule (3) pour la préparation des composés de formule (1)

7. Intermédiaires de formule générale (3) selon la revendication 5 dans lesquels l'atome de carbone stéréogénique de la chaîne propyle est de configuration absolue (S) .

## Claims

1. Process for the preparation of N-[3-[(2-methoxyphenyl)sulfanyl]-2-methylpropyl]-3,4-dihydro-2H-1,5-benzoxathiepin-3-amine of general formula (1) 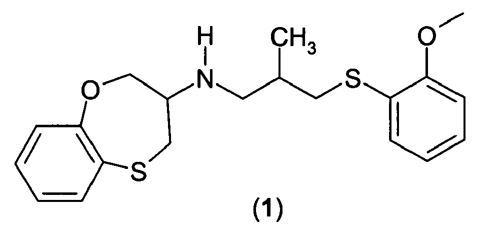 **characterised in that** it uses condensation of the intermediates of formulae (2) and (3) 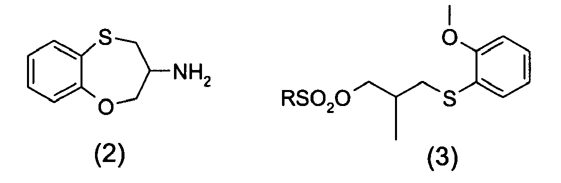 wherein the radical R is a methyl group or, preferably, a 4-methylphenyl group.

2. Process according to claim 1, **characterised in that** the intermediates of formula (3), wherein R is a methyl group or, preferably, a 4-methylphenyl group, 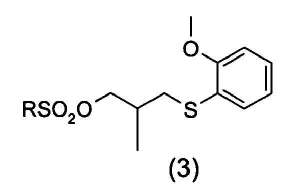 are prepared by using the reaction of 3-[(2-methoxyphenyl)sulfanyl]-2-methylpropanol 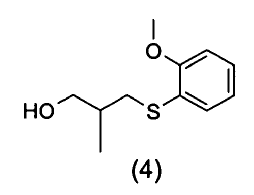 with an appropriate sulfonyl chloride to yield the compound of formula (3).

3. Process for the preparation of N-[3-[(2-methoxyphenyl)sulfanyl]-2-methylpropyl]-3,4-dihydro-2H-1,5-benzoxathiepin-3-amine of formula (1), according to claims 1 to 2, **characterised in that** the compound (1) is of absolute configuration (R) at the stereogenic carbon atom of the 3,4-dihydro-2H-1,5-benzoxathiepine fragment and of absolute configuration (S) at the stereogenic carbon atom of the propyl chain.

4. Process for the preparation of N-[3-[(2-methoxyphenyl)sulfanyl]-2-methylpropyl]-3,4-dihydro-2H-1,5-benzoxathiepin-3-amine of formula (1), according to claims 1 to 3, **characterised in that** the stereogenic carbon atom of the intermediate used of general formula (3) is of absolute configuration (S).

5. Intermediates of formula (3) wherein the radical R is a methyl group or, preferably, a 4-methylphenyl group

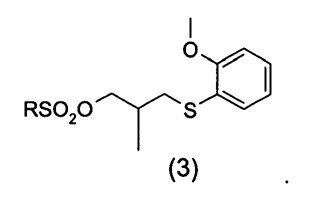

6. Use of intermediates of formula (3) in the preparation of compounds of formula (1).

7. Intermediates of general formula (3) according to claim 5, wherein the stereogenic carbon atom of the propyl chain is of absolute configuration (S).

## Patentansprüche

1. Verfahren zur Herstellung von N-(3-[(2-Methoxyphenyl)sulfanyl]-2-methylpropyl]-3,4-dihydro-2H-1,5-benzoxathiepin-3-amin der allgemeinen Formel (1) 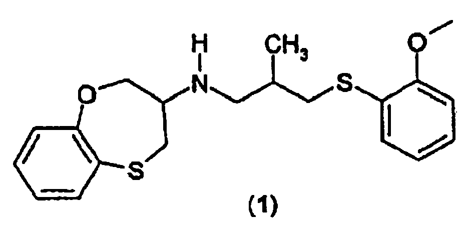 **dadurch gekennzeichnet, dass** die Zwischenprodukte der Formeln (2) und (3) 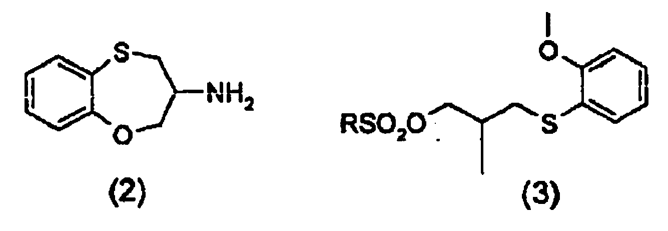 in welcher das Radikal R eine Methylgruppe oder, vorzugsweise, eine 4-Methylphenylgruppe ist, einer Kondensationsreaktion unterzogen werden.

2. Verfahren nach Anspruch 1, **dadurch gekennzeichnet, dass** die Zwischenprodukte der Formel (3), in welcher R eine Methylgruppe oder, vorzugsweise, eine 4-Methylphenylgruppe ist, 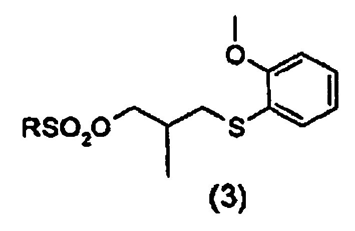 hergestellt werden, indem 3-[(2-Methoxyphenyl)sulfanyl]-2-methylpropanol 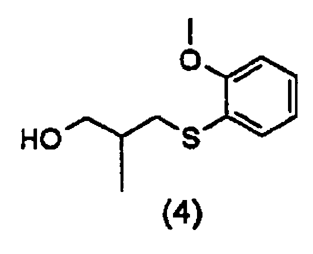 mit einem geeigneten Sulfonylchlorid umgesetzt wird, um die Verbindung der Formel (3) zu erhalten.

3. Verfahren zur Herstellung des N-(3-[(2-Methoxyphenyl)sulfanyl]-2-methylpropyl]-3,4-dihydro-2H-1,5-benzoxathiepin-3-amins der Formel (1), nach den Ansprüchen 1 bis 2, **dadurch gekennzeichnet, dass** die Verbindung (1) am asymmetrischen Kohlenstoffatom der 3,4-Dihydro-2H-1,5-benzoxathiepin-Gruppe eine absolute (R)-Konfiguration und am asymmetrischen Kohlenstoffatom der Propylkette eine absolute (S)-Konfiguration aufweist.

4. Verfahren zur Herstellung des N-(3-[(2-Methoxyphenyl)sulfanyl)-2-methylpropyl]-3,4-dihydro-2H-1,5- benzoxathiepin-3-amins der Formel (1) nach den Ansprüchen 1 bis 3, **dadurch gekennzeichnet, dass** das asymmetrische Kohlenstoffatom des verwendeten Zwischenprodukts der allgemeinen Formel (3) eine absolute (S)-Konfiguration aufweist.

5. Zwischenprodukte der Formel (3), in welcher das Radikal R eine Methylgruppe oder, vorzugsweise eine 4-Methylphenylgruppe ist.

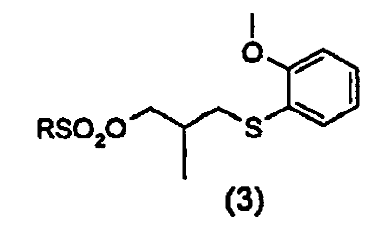

6. Verwendung der Zwischenprodukte der Formel (3) zur Herstellung von Verbindungen der Formel (1).

7. Zwischenprodukte der allgemeinen Formel (3) nach Anspruch 5, bei welchen das asymmetrische Kohlenstoffatom der Propylkette eine absolute (S)-Konfiguration aufweist.
